# EUROPEAN PATENT SPECIFICATION

(11) **EP 4 313 957 B1**
(45) Date of publication and mention of the grant of the patent: **07.05.2025**
(21) Application number: 22717163.4
(22) Date of filing: 23.03.2022
(51) Int. Cl.: C07D 257/02

(54) **PROCESS FOR THE PREPARATION OF CYCLEN**
VERFAHREN ZUR HERSTELLUNG VON CYCLEN
PROCÉDÉ DE PRÉPARATION DE CYCLÈNE

(30) Priority: 30.03.2021 EP 21165846
(43) Date of publication of application: 07.02.2024
(73) Proprietor: Bracco Imaging SPA, 20134 Milan (IT); Serichim Srl, 33050 Torviscosa Udine (IT)
(72) Inventor: BARATTA, Walter, 33100 Udine (IT); FIGLIOLIA, Rosario, 33100 Udine (IT); DELOGU, Pietro, 33050 Torviscosa (IT); ADAMO, Ilaria, 33050 Torviscosa (IT); BOCCALON, Mariangela, 34149 Basovizza (IT); BUONSANTI, Federica, 10010 Colleretto Giacosa (IT)
(74) Representative: Ravizza, Claudio
(86) International application number: PCT/EP2022/057614
(87) International publication number: WO 2022/207425

(56) References cited:
- CHUBURU FRANÇOISE ET AL: "Bis-Aminals of Linear Tetraamines: Kinetic and Thermodynamic Aspects of the Condensation Reaction", EUROPEAN JOURNAL OF ORGANIC CHEMISTRY, 26 February 2003 (2003-02-26), pages 1050 - 1055, XP055834489, Retrieved from the Internet <URL:https://chemistry-europe.onlinelibrary.wiley.com/doi/10.1002/ejoc.200390147> [retrieved on 20210824]
- WEISMAN GARY R ET AL: "A New Synthesis of Cyclen (1,4,7,10-Tetraazacyclododecane)", JOURNAL OF ORGANIC CHEMISTRY, 26 July 1996 (1996-07-26), pages 5186 - 5187, XP055834505, Retrieved from the Internet <URL:https://pubs.acs.org/doi/pdf/10.1021/jo9606665> [retrieved on 20210824], DOI: 10.1021/jo9606665
- HERVÉ G ET AL: "Condensation of Glyoxal with Triethylenetetraamine; Isomerization and Cyclization", EUROPEAN JOURNAL OF ORGANIC CHEMISTRY, WILEY-VCH, DE, vol. 2000, no. 1, 1 January 2000 (2000-01-01), pages 33 - 35, XP002740087, ISSN: 1434-193X, [retrieved on 19991213], DOI: 10.1002/(SICI)1099-0690(200001)2000:1<33::AID-EJOC33>3.0.CO;2-C
- YAMAMOTO HISASHI ET AL: "Regioselective carbonyl amination using diisobutylaluminum hydride", JOURNAL OF THE AMERICAN CHEMICAL SOCIETY, vol. 103, no. 14, 1 July 1981 (1981-07-01), pages 4186 - 4194, XP055834684, ISSN: 0002-7863, DOI: 10.1021/ja00404a035

## Description

### Technical field

The present invention relates to a new and straightforward process for the preparation of Cyclen.

Cyclen (1,4,7,10-tetraazacyclododecane) is a known compound largely used in the manufacturing of macrocyclic gadolinium complexes, which are widely employed as contrast agents in magnetic resonance imaging (MRI).

### Background of the invention

Several chemical processes are described in the literature for the preparation of Cyclen. Most of the reported synthetic routes are based on precursors of lower molecular weight that are suitably coupled to afford the macrocyclic ring and that differ on the methods used for the cyclization process.

Among them is the Richman-Atkins method (J. Am. Chem. Soc. 1974, 96, p. 2268-2270) that starts from diethylene triamine and diethanolamine. The two starting materials, where the amino and hydroxyl groups are suitably protected, are first coupled to yield the tetraazacyclododecane ring then deprotected to obtain free Cyclen.

As an additional example, the Schering US 5,744,616 patent discloses the tetramerization of protected ethanolamine via the corresponding protected aziridine which, after cyclization, is deprotected to yield Cyclen.

Alternative preparation methods are also known as employing triethylene tetramine (TETA) as starting material, first reacted with suitable dicarbonyl derivatives. The main synthetic steps include a first reaction with a dicarbonyl reagent to protect the amino groups other than the terminal ones that are then alkylated by an ethylene substituted reagent, for instance 1,2-dibromoethane. The cyclization reaction thus affords a tetracyclic intermediate which is finally deprotected to give Cyclen; see, as an example, the patents or patent applications WO 00/53588, US 6156890, WO 96/28432 and US 5587451.

All of these routes are usually characterized by low atomic efficiencies and cumbersome synthetic steps. In addition, the use and/or formation of hazardous compounds, i.e. aziridines, represents a major drawback for large scale industrial production.

A simpler approach was described by Weisman and Reed [J. Org. Chem. 1996, 61, 5186-5187 and Organic Syntheses, Coll. Vol. 10, p.667 (2004*); Vol. 78, p.73 (2002)*]*,* starting from dithiooxamide and triethylene tetramine. Dithiooxamide acts as a templating agent for the additional ethylene bridging moiety that is needed to properly close the tetrameric ring. Then, Cyclen is finally obtained by reduction of the corresponding tricyclic bis-amidino derivative with diisobutyl aluminium hydride (DIBAL-H).

This route includes only two synthetic steps with high atomic efficiency. However, it presents the major drawback of using dithiooxamide, a quite expensive raw material further leading to the formation of ammonia and also of hydrogen sulfide as highly toxic by-product. This procedure, therefore, is not or hardly to be intended for the industrial production of Cyclen.

More recent attempts for Cyclen preparation are also reported where dicarbonyl derivatives alternative to dithiooxamide were used. To this extent, following the aforementioned synthetic scheme proposed by Reed, the patent application KR20190108383 discloses a method for the preparation of Cyclen comprising the use of oxamide in place of dithiooxamide, so as to obtain the tricyclic bis-amidino derivative finally reduced to Cyclen in the presence of lithium aluminium hydride. Nevertheless, the above method implies the release of gaseous ammonia as a byproduct and, remarkably, is further characterized by very poor yields in the final product.

### Description of the invention

The known drawbacks associated to the former manufacturing methods for Cyclen preparation are overcome by the process object of the present invention.

The present invention thus refers to a process for the preparation of Cyclen which process comprises:
a) reacting glyoxal with triethylene tetramine so as to obtain decahydrodiimidazo[1,2-a:2',1'-c]pyrazine; and
b) reducing the thus obtained decahydrodiimidazo[1,2-a:2',1'-c]pyrazine with a reducing agent selected from the group consisting of dialkyl aluminium hydride or trialkylamine alane complex, or mixtures thereof;
wherein the above alkyl groups, the same or different in each occurrence, are selected from straight or branched C₁-C₆ alkyl groups.

For a better understanding of the reaction steps within the process of the invention see Scheme 1 below: 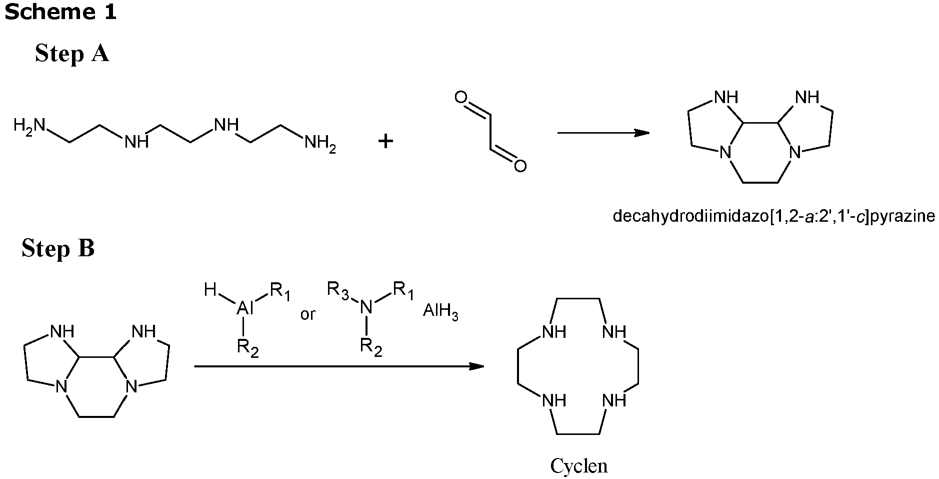 wherein R₁, R₂ and R₃, within the dialkyl alluminium hydride or trialkylamine alane complex, have the above reported meanings.

The process of the invention is particularly advantageous as it avoids the use and/or release of hazardous materials during the whole course of the reactions and further provides for the synthesis of Cyclen in high yields and purity, by efficiently operating under mild conditions.

The starting materials and all of the selected reagents are known and easily commercially available. Glyoxal, in particular, is much cheaper and certainly safer than dithiooxamide being used according to the aforementioned prior art processes.

Overall, and as better set forth below, the process of the invention appears to be particularly attractive for large scale industrial production.

### Detailed description of the invention

The reaction of triethylene tetramine with glyoxal is known in the art as reported, for instance, by Hervè et al. [Tetrahedron Letters 40 (1999) 2517-2520] and leads to the formation of a mixture of four isomers which structures are reported below:

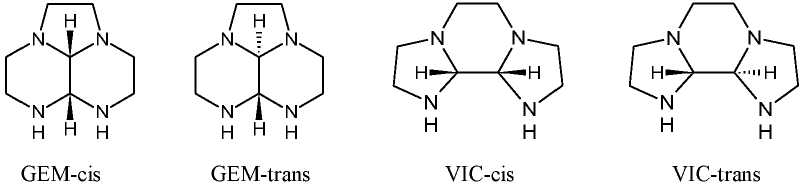

The percentage of the said isomers, namely the composition of the resulting mixture, strongly depends on the adopted reaction conditions that may lead to the two couples of tricyclic derivatives having geminal (GEM) conformation, known to be thermodynamically favored, or vicinal (VIC) conformation, known to be kinetically preferred, each one in cis and trans configuration.

To this extent, however, it is worth pointing out that the VIC isomers are the sole useful compounds that, by reduction according to the process of the invention, allow for the preparation of Cyclen.

Conversely, and as further detailed in the experimental section, the corresponding reduction of the GEM isomers may only lead to the formation of a piperazine by-product (see structure below)

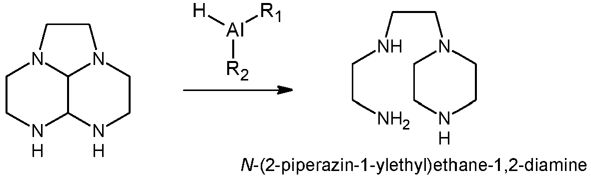

As such, especially if the GEM isomers are preponderant or even present in significant amounts in the VIC/GEM mixture undergoing the subsequent reductive step, the obtained piperazine derivative may render much more difficult the final recovery and purification of Cyclen.

For all of the above reasons it is of paramount importance to rely on a process for Cyclen manufacturing that maximizes the yields in the VIC isomers.

To this extent, some efforts to obtain VIC rich mixtures are reported in the art.

As an example, Chuburou et al. (Eur. J. Org. Chem. 2003, 1050-1055) disclosed the formation of the VIC isomers exclusively by operating in anhydrous conditions, that is by using ethanolic solutions of both glyoxal and triethylene tetramine as starting materials, at -10°C for 2 hours. However, this methodology is not applicable to the industrial scale, at least once starting from commercial glyoxal that is indeed available as an aqueous solution (maximum concentration of 40% in water), so that the presence of water cannot be avoided.

In the alternative, the treatment of aqueous glyoxal with triethylene tetramine in ethanol, at room temperature and under overnight stirring, afforded the VIC isomers in about 75-80%, as reported in the patent application WO 96/28432, even if no information are provided on the actual composition of the mixture.

Similar operating conditions for glyoxal and triethylene tetramine coupling but even requiring a prolonged reaction time of 20 hours, are also disclosed in the patent application CA 2353680, though data on the composition of the isomers mixture are still missing.

Along these lines however, Argese et al. [Tetrahedron 62 (2006) 6855-6861] disclosed that by operating under the conditions reported in the prior art, more specifically according to the methodology disclosed in WO 96/28432, the initial reaction mixture containing an excess of VIC isomers changed its composition during the workup, so as to lead to a mixture of GEM and VIC isomers.

In particular, by analyzing the reaction mixture after only 0.5 hours through a micellar electrokinetic chromatographic method (MEKC), a mixture of VIC isomers was formed with no detection of the GEM ones, hence confirming that the former are the kinetically preferred reaction products.

Then, during workup and collection of the isolated product, a partial isomerization to GEM isomers was observed, hence leading to the aforementioned drawbacks that may be encountered during the subsequent reduction step on the undesired GEM isomers precursors.

Therefore, it is not only of paramount importance to rely on a process for Cyclen manufacturing being highly selective towards the VIC isomers but, also, on a process that minimizes the rate of conversion of the VIC isomers to the more stable GEM ones, during the workup phase.

All of the above is best accomplished by the present invention that provides for a very efficient process to obtain a convenient feed of VIC isomers for the subsequent chemical step of reduction, by starting from commercially available aqueous solutions of glyoxal. The said process allows for a selectivity high as 85% or more in the VIC isomers, without any significant conversion of these latter to the GEM ones during the workup phase.

For the avoidance of doubts, the herewith referred VIC isomers are the cis and trans isomers of the bis-aminals referred to as decahydrodiimidazo[1,2-a:2',1'-c]pyrazine being obtained at the end of step (a) of the process.

Therefore, unless otherwise provided, the above terms/terminologies may be used interchangeably in the present description.

In practical terms, and to avoid as much as possible the above prior art drawbacks, the commercially available aqueous solution of glyoxal, more specifically the 40% aqueous solution of glyoxal, is slowly added to the anhydrous ethanolic solution of the amine, for instance continuously or portionwise, by operating according to conventional means.

Preferably, the said glyoxal addition is carried out in a time period ranging from at least 5 minutes to at least 40 minutes. Even more preferably, the addition is carried out in a time period ranging from at least 10 minutes to at least 35 minutes.

After the addition of the aqueous solution of glyoxal is ended, the reaction is then brought to completion in a short period of time, for instance of from about 5 to about 30 minutes.

Overall, the reaction according to step (a) of the process is best accomplished in a time period of about 1 hour or less, for instance in a time period of from about 30 minutes to about 60 minutes, being this period comprehensive of both glyoxal addition and reaction completion.

It is thus a further object of the invention a process for the preparation of Cyclen wherein step (a) is carried out by adding an aqueous solution of glyoxal to a solution of the amine in anhydrous ethanol, and by carrying out the reaction in a time period of about 1 hour or less.

According to an additional embodiment of the invention, the reaction of step (a) is carried out below room temperature. The above conditions may suitably apply during the addition of glyoxal to the ethanolic solution of the amine and also during the course of the reaction up to completion.

It is therefore a further object of the invention a process for the preparation of Cyclen wherein step (a) is carried out by adding an aqueous solution of glyoxal to a solution of the amine in anhydrous ethanol, kept below room temperature, and by carrying out the reaction below room temperature.

Preferably, the temperature is in the range of from about 0°C to about 20°C and, even more preferably, from about 0°C to about 10°C.

From all of the above, it is clear to the skilled person that any of the preferred operative conditions of temperature and reaction time may be properly combined.

Therefore, it is a further preferred embodiment of the invention a process for the preparation of Cyclen wherein step (a) is carried out by adding an aqueous solution of glyoxal to a solution of the amine in anhydrous ethanol, kept below room temperature, and by carrying out the reaction below room temperature and in a time period of about 1 hour or less.

Unlike what it is reported in the prior art, the process of the invention enables for the preparation of the VIC isomers with high yields and selectivity by operating under mild operative conditions and in a relatively short period of time. As such, by taking advantage of the short residence time that is required for the coupling reaction according to step (a), the process of the invention can be conveniently operated either in semi-batch or even in a continuous mode.

From the above it is clear to the skilled person that as per step (a), the addition of glyoxal to triethylene tetramine, when each of them is properly dissolved in a solvent medium, may be also achieved through the contact or by contacting the former component with the second, as per the terminology typically adopted in continuous mode or flow chemistry techniques where streams of reactants are suitably contacted so as to carry out the desired reactions.

Therefore, unless otherwise provided in the present description, the terms "adding" or "addition" or even "additions", once used in the context of adding a reactant to another, may be conveniently replaced by the terms "contacting" or "contact" or "contacts", as the case may be, eventually more appropriate in continuous mode processes.

Examples of reactors and techniques that could be employed according to the invention, once step (a) of the process is operated under continuous mode, are the known reactors and techniques that may comprise the continuous feeding of the reactants to any suitable reactor with continuous formation and exiting of the reaction product stream. Non limiting examples among them are, for instance, tubular reactors otherwise referred to as pipe reactors, plug flow reactors, microreactors, continuous stirred tank reactors (CSTRs), and the like.

Any parameter of the process for instance including streams flow rate, residence time and temperature, may be then properly selected according to conventional means so as to fit with the desired reaction conditions to be met.

As an example, step (a) of the process of the invention may be easily carried out in a continuous mode by operating through a CSTRs cascade properly fed with suitable solutions of triethylene tetramine in anhydrous ethanol and aqueous glyoxal. Feeding of both streams of reactants may be separately operated by means of conventional syringe pumps or through any suitable pumping system.

Preferably, the ethanolic solution of the amine is fed to a first CSTR, while the proper amount of aqueous glyoxal solution may be then distributed along the CSTRs cascade.

Likewise, by operating in any tubular reactor, the aqueous glyoxal stream may be conveniently distributed among a plurality of inlets positioned along the length of the reactor itself previously fed with the ethanolic solution of the amine.

From the above, it is thus clear to the skilled person that a proper geometry of the reactor together with any selected feeding rate for each of the flow streams may thus provide for an optimal admixing of the reactants for any selected residence time. Temperature, like any other parameter of the process, may be then suitably controlled according to conventional means so as to fit with the desired conversion of the starting materials and the subsequent collection of the VIC isomers.

It is thus a further object of the invention a process for the preparation of Cyclen wherein step (a) is carried out in a continuous mode by contacting an aqueous solution of glyoxal to an ethanolic solution of the amine.

Preferably, the addition of the aqueous solution of glyoxal is carried out portionwise, along the reactor length of any given tubular reactor or anyhow distributed among a plurality of continuous reactors, for instance within a CSTRs cascade.

The subsequent step (b) of the process of the invention implies the reduction of the VIC bis-aminals, that is of decahydrodiimidazo[1,2-a:2',1'-c]pyrazine.

The reaction is carried out in homogeneous phase by treatment of the above VIC bis-aminals with the selected reducing agent, in the presence of hydrocarbon solvents at refluxing conditions.

As such, at the end of step (a) a solvent change is carried out.

This operation can be accomplished according to conventional means, either in batch, semi-batch or preferably in a continuous mode, by feeding the reaction crude coming from step (a) and the selected water immiscible hydrocarbon solvent to a distillation column.

Ethanol and water may be thus collected as top products whilst the VIC bis-aminals in the anhydrous hydrocarbon solvent are recovered at the bottom.

The distillation is preferably carried out under vacuum and typically occurs in few minutes only so as to provide for a VIC-isomers rich hydrocarbon stream, either directly intended for the subsequent reduction step or to be eventually worked-up for products collection. In both cases, however, no or minimal conversion to the GEM isomers could be observed.

Suitable hydrocarbon solvents are all of the solvents that may enable for the collection of ethanol and water as top products whilst remaining at the bottom of the distillation column.

Typical examples may thus include aromatic or aliphatic hydrocarbons such as toluene, xylene, ethylbenzene, mesitylene, hexane, heptane, octane, isooctane, nonane, decane and the like, or even mixtures thereof.

Preferably, the hydrocarbon solvent is selected from toluene, xylene, ethylbenzene, mesitylene or mixtures thereof. Even more preferably the hydrocarbon solvent is toluene.

As far as the reducing agent is concerned, any of R₁, R₂ and R₃ within the above dialkyl aluminium hydrides AlHR₁R₂ or trialkylamine alane AlH₃·NR₁R₂R₃ complexes represent, each independently, a straight or branched C₁-C₆ alkyl group. Non limiting examples may thus include methyl, ethyl, n-propyl, isopropyl, n-butyl, isobutyl, sec-butyl, tert-butyl, n-pentyl, n-hexyl and the like.

More preferably, the said R₁, R₂ and R₃ are, each independently, straight or branched C₁-C₄ alkyl groups.

According to a preferred embodiment of the invention, the reducing agent is selected from the group consisting of diisobutyl aluminium hydride (DIBAL-H), dimethylethylamine alane complex, or mixtures thereof.

Even more preferably the reducing agent is diisobutyl aluminium hydride.

The said reducing agents are either commercially available, for instance in toluene solution, or anyhow preparable according to known methods.

For a general reference to the preparation of the dialkyl aluminium hydrides see, as an example, T. Wartik and H. I. Schlesinger (J. Am. Chem. Soc. 1953, 75, 835-839), H. Lehmkuhl, K. Ziegler (Methoden Org. Chem. (Houben-Weyl), 4th ed., Vol. XIII/4, Thieme, Stuttgart, 1952, p. 58), and J. J. Eisch (Organometallic Syntheses 1981, vol 2, p. 136, Academic Press: New York).

For a general reference to the preparation of the alane complexes see, as an example, T. D. Humpries et al. (Dalton Transaction 2013, 42, 6965-6978), E. M. Marlett and W. S. Park (J. Org. Chem. 1990, 55, 2968-2969), and R. A. Kovar et al. (Inorg. Synth. 1977, 17, 36-42).

The reduction reaction requires 4 equivalents of reducing agent and occurs in two stages: a first portion of reducing agent is in fact needed to substitute each of the hydrogen atoms of the secondary amino groups within the decahydrodiimidazo[1,2-a:2',1'-c]pyrazine, whilst a second portion of reducing agent provides for ring reduction, thus affording the macrocylic tetraazacyclododecane structure wherein the nitrogen atoms are still bound to the aluminium groups (i.e. >N-AlR₁R₂). Then, as set forth below, subsequent workup according to known methods finally provides for Cyclen recovery.

The said portions of any selected reducing agent according to the invention may be either combined or alternatively separated over the time, so as to comprise a first portion of reducing agent then followed by a second portion of the same or even different reducing agent, in both cases added to the substrate undergoing the reductive step.

At the end of the addition, reaction completion is typically carried out under refluxing conditions at atmospheric pressure.

Therefore, it represents a further object of the invention a process for the preparation of Cyclen wherein step (b) comprises:
(b') adding the selected dialkyl aluminium hydride or trialkylamine alane complex or mixtures thereof to a solution of decahydrodiimidazo[1,2-a:2',1'-c]pyrazine in a hydrocarbon solvent; and then
(b") refluxing the reaction mixture in that selected hydrocarbon solvent.

Preferably, the addition of the reducing agent in step (b') is carried out according to conventional means, for instance dropwise or portionwise, at a temperature in the range of from about 0°C to about 50°C and, even more preferably, at room temperature.

On the other side, refluxing under step (b") is carried out at a temperature of about 100°C to about 170°C, depending on the selected hydrocarbon solvent to be used, for a time varying from a few hours to few days, for instance from about 18 hours to about 96 hours, depending on the selected operative conditions and choice of reducing agent being employed.

As an example, in fact, the use of DIBAL-H enabled for the quantitative formation of the macrocyclic derivative (i.e. Cyclen precursor) by refluxing the reaction mixture in the presence of toluene, that is at about 110°C at atmospheric pressure, in a period of from about 18 hours to about 40 hours, more preferably from about 20 hours to about 24 hours.

Surprisingly, we have now found that the reaction time in step (b") can be dramatically shortened, being for instance comprised from about 2 to about 12 hours and even more preferably from about 2 to about 6 hours, whilst maintaining proper selectivity and high yields. The above was in fact achieved by refluxing the reaction mixture under pressure. Therefore, it represents a further object of the invention a process for the preparation of Cyclen wherein step (b) comprises:
(b') adding the selected dialkyl aluminium hydride or trialkylamine alane complex or mixtures thereof to a solution of decahydrodiimidazo[1,2-a:2',1'-c]pyrazine in a hydrocarbon solvent; and then
(b") refluxing the reaction mixture in that selected hydrocarbon solvent under pressure.

In this latter occurrence, step (b") may be easily accomplished according to conventional means in any suitable reactor intended to be operated under pressure, for instance of from about 1 to about 6 atmospheres. Proper pressure conditions can be easily achieved depending on the selected hydrocarbon solvent and the refluxing temperature being adopted, for instance comprised from about 120°C to about 160°C.

Preferably, as said, step (b") is carried out in toluene by operating under pressure, at a temperature of from about 120°C to about 160°C, for instance at about 140°C, for a time period of from about 2 to about 6 hours, for instance in a time period of about 4 hours.

Finally, the isolation of Cyclen may be carried out according to known procedures, for instance as described by Yamamoto and Reed (J. Am. Chem. Soc., Vol. 103, No. 14, 1981, p. 4186-4194 and J. Org. Chem. 1996, 61, 5186-5187) based on the workup procedure for reactions involving DIBAL-H as reducing agent (Fieser, L. F.; Fieser, M. Reagents for Organic Synthesis; John Wiley and Sons: New York, 1967; Vol. 1, p. 260). To this extent, hydrolysis of the Al-isobutyl species within the macrocyclic derivative obtained at the end of step (b) of the process with water and treatment with NaF or NaOH affords the precipitation of aluminium as fluoride or hydroxide species. Final extraction with a suitable solvent, for instance toluene-chloroform mixtures, and subsequent purification of the resulting product gives Cyclen in 80-90 % isolated yield referred to the VIC isomers present in the bis-aminals feed.

From all of the above it is clear to the skilled person that any of the preferred embodiments of the invention and that may either refer to steps (a) or (b) of the process may be suitably combined to each other. As an example, any of the parameters of the process for instance including the time of addition of the aqueous solution of glyoxal to the ethanolic solution of the amine and the time to reaction completion, as per step (a), together with the selected reaction temperature may be suitably combined. Likewise, the above may also apply to the choice of the hydrocarbon solvent, the selected reducing agent, and any of the parameters referring to step (b) of the process, for instance including temperature and/or pressure conditions being applied.

Additional embodiments of the inventions are also represented by each of the above steps (a) and (b) of the process per se, together with any of the aforementioned preferred operative conditions.

In particular, it is a further object of the invention a process for the preparation of decahydrodiimidazo[1,2-a:2',1'-c]pyrazine which process comprises adding an aqueous solution of glyoxal to a solution of triethylene tetramine in anhydrous ethanol, and by carrying out the reaction in a time period of about 1 hour or less.

It is a further object of the invention a process for the preparation of Cyclen which process comprises adding a reducing agent selected from the group consisting of dialkyl aluminium hydride or trialkylamine alane complex, or mixtures thereof, to a solution of decahydrodiimidazo[1,2-a:2',1'-c]pyrazine in a hydrocarbon solvent, so as to obtain the 1,4,7,10-tetraazacyclododecane derivative wherein the nitrogen atoms are still bound to the dialkyl aluminium groups, then converted to Cyclen according to known methods; wherein the above alkyl groups, the same or different in each occurrence, are selected from straight or branched C₁-C₆ alkyl groups.

Finally, as the aforementioned N-(2-piperazin-1-ylethyl)ethane-1,2-diamine is a known compound that finds a variety of applications, for instance in the field of resins and polymers as curing additive, the process for its preparation through the reduction of the above GEM derivative with trialkyl aluminium hydrides or trialkylamine alane complexes, represents a further object of the invention.

Further details concerning the process of the invention are reported in the following experimental section, with the sole aim to better illustrate it without posing any limitation.

### Example 1

### Fed-batch synthesis of decahydrodiimidazo[1,2-a:2',1'-c]pyrazine (i.e. VIC-bisaminals)

Di-hydrated triethylene tetramine (30 grams, 164.6 mmoles) and 600 mL of anhydrous ethanol were charged to a three necks 1 liter round bottom flask with a stirrer. The solution was cooled to 5°C and glyoxal 40% in water (24 grams, 164.6 mmoles) was slowly added over 35 minutes. The solution was aged for 10 minutes and fed to the bottom of a 5 trays distillation column containing 150 grams of toluene. Distillation was operated under vacuum, with a bottom temperature equal to 35-40°C, withdrawing the aqueous/ethanolic phase at the top of the column using a Dean-Stark separator. After residual solvent removal 29 grams of a cereous solid were obtained, having a VIC/GEM ratio 6.1/1 (86 % VIC selectivity, 83% chromatographic yield).

### Example 2

### Fed-batch synthesis of decahydrodiimidazo[1,2-a:2',1'-c]pyrazine (i.e. VIC-bisaminals)

Di-hydrated triethylene tetramine (10.3 grams, 56.3 mmoles) and 205 mL of anhydrous ethanol were charged to a three necks 1 liter round bottom flask with a stirrer. The solution was cooled to 5°C and glyoxal 40% in water (8.18 grams, 56.3 mmoles) was slowly added in 10 minutes. The solution was aged for 30 minutes and then concentrated under vacuum to a volume of 14 mL. The concentrated solution was fed to the bottom of a 5 trays distillation column containing 60 grams of toluene. Distillation was operated under vacuum, with a bottom temperature equal to 35-40°C, withdrawing the aqueous/ethanolic phase at the top of the column using a Dean-Stark separator. After residual solvent removal 10 grams of a cereous solid were obtained, having a VIC/GEM ratio 6.7/1 (87 % VIC selectivity, 84% chromatographic yield).

### Example 3

### Continuous synthesis of decahydrodiimidazo[1,2-a:2',1'-c]pyrazine

When step (a) of the process is carried out in a continuous mode and each of the two streams of reactants, namely the 40% aqueous solution of glyoxal and triethylene tetramine in anhydrous ethanol, are fed through an inlet of a tubular reactor, the reaction kinetics may simulate the one occurring in a pure batch reactor. However, as this approach gave poor results affording a VIC selectivity as low as 65%, a simulation model was carried out for the continuous synthesis of the title compound through a CSTRs cascade.

More in particular, this simulation model represents the continuous reactor as a CSTRs cascade where the triethylene tetramine ethanolic solution is fed to a first cell, while the 40% aqueous glyoxal solution is fed distributing the total quantity in a number of parts equal to the number of CSTR stages, and feeding each part sequentially to each stage. The VIC bisaminals synthesis was thus simulated in a CSTRs cascade including 10 stages, each one having a volume of 20 mL for a total volume of 200 mL. A solution of triethylene tetramine with a concentration of 0.317 moles/L in anhydrous ethanol is fed to the first stage at a rate of 0.04 L/min. A 40% aqueous solution of glyoxal is fed to each stage at a rate of 0.145 mL/min per stage. Temperature is of 25°C and overall residence time is of 5 minutes.

From the simulation model analysis it can be observed that by distributing the aqueous glyoxal solution stream along the reactor length the selectivity towards the VIC isomers can be surprisingly increased up to 80% and higher, with a final triethylene tetramine conversion of 94%. The model predicts that the VIC selectivity in the stream exiting the last CSTR increases with the number of CSTRs forming the reactor, as per the following table:

| **n° of CSTRs** | **VIC selectivity** |
|---|---|
| 1 | 64.22% |
| 3 | 76.49% |
| 5 | 78.72% |
| 8 | 79.93% |
| 10 | 80.33% |

### Example 4

### Reduction of bis-aminal mixture (VIC/GEM = 5) with DIBAL-H

The bisaminal mixture (1 g, 5.94 mmol, VIC/GEM =5, VIC = 83%) was placed in a 100 mL Schlenk tube and subjected to 3 vacuum-argon cycles and placed in a cold water bath (~8°C). Then, 4.5 equivalents of a 1 M solution of DIBAL-H in toluene (26.75 mL, 26.75 mmol) were added dropwise under argon flow. After formation of a clear light yellow solution the Schlenk was placed in an oil bath and stirred at reflux temperature for 40 hours. The solution was allowed to cool down to room temperature and NaF (4.54 g, 108 mmol, 18 equiv.) was added, obtaining a suspension which was stirred for 10 minutes in an ice bath. Water 1.62 mL (90 mmol, 15 equiv.) was added dropwise over 5 minutes time. The slurry was heated up to 75°C and stirred for 30 minutes and filtered through celite pad. The cake was washed with hot toluene (3x20 mL) and EtOH (2x10 mL) which were removed in vacuum, affording 900 mg of a white product (88 % yield). The ¹³C{¹H} NMR analysis of the crude product gave 85% of Cyclen (δ_{C} = 45.9 ppm) and 15% of N-(2-piperazin-1-ylethyl)ethane-1,2-diamine.

### Example 5

### Reduction of bisaminal mixture (VIC/GEM = 5) with DIBAL-H, 10 g scale

A bisaminals mixture (10 g, 59.4 mmol, VIC/GEM = 5, VIC = 83%) was placed in a 500 mL round bottle flask and subjected to 3 vacuum-argon cycles. Then 4.5 equivalents of a 1 M solution of DIBAL-H in toluene (267.5 mL, 267.5 mmol) were added portionwise under argon flow. After complete dissolution of the mixture the flask was placed in an oil bath removing the overpressure generated during the heating up and mechanically stirred at reflux for 24 hours. The solution was allowed to cool down at room temperature, NaF (45.4 g, 1080 mmol. 18 equiv.) added and the stirred for 10 minutes. The flask was placed in an ice bath and water 16.2 mL (900 mmol, 15 equiv.) was added dropwise over 1 hour. The slurry was stirred for 15 minutes at 70°C and filtered with a glass funnel filter. The cake was washed with hot toluene (3x50 mL), EtOH (2x50 mL) and the solvents were removed in vacuum, affording 7 g of a white product (68 %). The ¹³C{¹H} NMR analysis of the crude product gave 80% of Cyclen and 20% of N-(2-piperazin-1-ylethyl)ethane-1,2-diamine.

### Example 6

### Reduction of bisaminal mixture (VIC/GEM = 5) with DIBAL-H, under pressure at 140°C

A bisaminals mixture (336 mg, 2 mmol, VIC/GEM = 5, VIC = 83%) was placed in a 25 mL Schlenk tube and subjected to 3 vacuum-argon cycles. Then 4.5 equivalents of a 1 M solution of DIBAL-H in toluene ((9 mL, 9 mmol) were added dropwise under argon flow. After complete dissolution, the mixture was transferred under argon with a canula into a sealed glass reactor, placed in an oil bath and stirred at 140°C for 4 hours. The solution was allowed to cool down at room temperature and NaF (1.55 g, 36 mmol, 18 equiv.) was added. The suspension was placed in a cold water bath (~8 °C) and water 480 µL (27 mmol, 13 equiv.) was carefully added dropwise. The slurry was stirred for 15 minutes at 70°C and filtered with a glass funnel filter. The cake was washed with hot toluene (3x10 mL), EtOH (2x5 mL) and the solvents were removed in vacuum, affording 296 mg of a white product (86 % yield). The ¹³C{¹H} NMR analysis of the crude product gave 91% of Cyclen and 9% of N-(2-piperazin-1-ylethyl)ethane-1,2-diamine.

### Example 7

### Reduction of bisaminal mixture (GEM/VIC = 5) with DIBAL-H

A bisaminals mixture (168 mg, 1 mmol, GEM/VIC = 5, GEM = 83 %) was placed in a 100 mL Schlenk tube and subjected to 3 vacuum-argon cycles. Then 4.5 equivalents of a 1 M solution of DIBAL-H in toluene (4.5 mL, 4.5 mmol) were added dropwise. After complete dissolution of the mixture the Schlenk was placed in oil bath removing the overpressure generated during the heating up and stirred at reflux. After 40 hours the solution was allowed to cool down at room temperature and NaF (755 mg, 18 mmol, 18 equiv.) was added and the suspension was stirred for 10 minutes. The Schlenk was placed in a cold water bath (~8 °C) and water 240 µL (13.3 mmol, 13 equiv.) was carefully added dropwise. The slurry was stirred for 15 minutes at 70 °C and filtered with a glass funnel filter. The cake was washed with hot toluene (3x5 mL), EtOH (2x5 mL) and the solvents were removed in vacuum, affording 150 mg of a white product, (87 % Yield). The ¹³C{¹H} NMR analysis of crude product gave 80% of N-(2-piperazin-1-ylethyl)ethane-1,2-diamine (δ_{C} = 58.4, 54.4, 52.6, 46.0, 45.9, 41.6 ppm) and 20% of Cyclen.

### Example 8

### Synthesis of Cyclen

30g of triethylene tetramine dihydrate (165 mmol) and 600 mL of ethanol were introduced into a 500 mL flask to obtain a clear colourless solution. The solution was cooled with an ice bath up to 5°C, then 24 g of 40% solution of glyoxal in water (165 mmol) were added in 35 minutes. The reaction solution was kept at 5°C for 10 minutes. The clear yellowish solution was concentrated under reduced pressure at 50°C to remove the solvent, affording 33g of clear orange liquid were obtained.

Toluene (150 g) was added and the mixture was poured into a 250 mL three-necked flask equipped with a 5-plate column, heavy phase collection head and condenser. The system was connected to vacuum and heated to 35-40°C in an oil bath (plate set 45°C, Tout 38°C, Tin 35°C) and the distillate was taken and discarded. At the end of the distillation the solvent was completely roto-evaporated, obtaining a waxy yellowish solid (29 g) containing 83.8% of the VIC isomers (chromatographic analysis).

10 g of the obtained bisaminals (59.4 mmol) and 20 mL of toluene were introduced into a 500 mL three-necked flask equipped with a magnetic stir bar and dropping funnel, obtaining a clear yellow solution. Internal temperature was 28°C. By means of a cannula, under nitrogen pressure, 180 mL of DIBAL-H 25% in toluene (268 mmol) were poured into the dropping funnel, then at room temperature the toluene solution of DIBAL-H was slowly added (1.5 hours) while maintaining the nitrogen atmosphere (inlet nitrogen above the dripping funnel). Initially an exothermy was noted: the temperature rose to 40°C and the system was cooled with an ice bath. The exothermy ended after the addition of the first 70 mL of the solution. At the end of the addition, the dropping funnel was removed and replaced with a reflux refrigerant. The system was kept at reflux (set 120°C) for 20 h. A 1 mL analytical sample treated with NaF and ethanol afforded by chromatography a conversion of bisaminals of 96% and a reduction yield to Cyclen equal to 80%. The overall yield of Cyclen on triethylene tetramine resulted 70%.

### Example 9

### Reduction of bisaminal mixture (VIC/GEM = 5) with DIBAL-H and alane N,N-dimethylethylamine complex solution

A bisaminals mixture (168 mg, 1 mmol, VIC/GEM = 5, VIC = 83%) was placed in a 25 mL Schlenk tube and subjected to 3 vacuum-argon cycles. Then 2.1 equivalents of a 1 M solution of DIBAL-H in toluene (2.1 mL, 2.1 mmol) were added dropwise under argon flow. After complete dissolution, 1.5 equivalents of a 0.5 M alane N,N-dimethylethylamine complex (NMe₂Et·AlH₃) solution in toluene (3 mL, 1.5 mmol) were added. The Schlenk was placed in an oil bath removing the overpressure generated during the heating up and stirred at reflux for 96 h. The solution was allowed to cool down at room temperature and NaF (755 mg, 18 mmol, 18 equiv.) was added and the suspension was stirred for 10 min. The Schlenk was placed in a cold water bath (~8 °C) and water 240 µL (13.3 mmol, 13 equiv.) was carefully added dropwise. The slurry was stirred for 15 min at 70°C and filtered with a glass funnel filter. The cake was washed with hot toluene (3x50 mL), EtOH (2x50 mL) and the solvents were removed in vacuum, affording 147 mg of a white product (85% Yield). The ¹³C{¹H} NMR analysis of the crude product gave 83% of Cyclen and 17% of N-(2-piperazin-1-ylethyl)ethane-1,2-diamine and VIC and GEM starting products.

## Claims

1. A process for the preparation of Cyclen which process comprises:
a) reacting glyoxal with triethylene tetramine so as to obtain decahydrodiimidazo[1,2-a:2',1'-c]pyrazine; and
b) reducing the thus obtained decahydrodiimidazo[1,2-a:2',1'-c]pyrazine with a reducing agent selected from the group consisting of dialkyl aluminium hydride, trialkylamine alane complex, or mixtures thereof;
wherein the above alkyl groups, the same or different in each occurrence, are selected from straight or branched C₁-C₆ alkyl groups, and
wherein step (a) is carried out by adding an aqueous solution of glyoxal to a solution of the amine in anhydrous ethanol, and by carrying out the reaction in a time period of 1 hour or less.

2. A process according to claim 1 wherein step (a) is carried out by adding an aqueous solution of glyoxal to a solution of the amine in anhydrous ethanol, kept below room temperature, and by carrying out the reaction below room temperature.

3. A process according to claim 2 wherein the temperature is in the range of from 0°C to 10°C.

4. A process according to anyone of claims from 1 to 3 wherein, at the end of step (a), a solvent change is operated comprising feeding the reaction crude from step (a) and a hydrocarbon solvent to a distillation column.

5. A process according to claim 4 wherein the hydrocarbon solvent is selected from the group consisting of toluene, xylene, ethylbenzene, mesitylene, hexane, heptane, octane, isooctane, nonane, decane or mixtures thereof.

6. A process according to claim 5 wherein the hydrocarbon solvent is toluene.

7. A process according to claim 1 wherein step (b) comprises:
(b') adding the selected dialkyl aluminium hydride or trialkylamine alane complex or mixtures thereof to a solution of decahydrodiimidazo[1,2-a:2',1'-c]pyrazine in a hydrocarbon solvent; and then
(b") refluxing the reaction mixture in that selected hydrocarbon solvent at atmospheric pressure.

8. A process according to claim 1 wherein step (b) comprises:
(b') adding the selected dialkyl aluminium hydride or trialkylamine alane complex or mixtures thereof to a solution of decahydrodiimidazo[1,2-a:2',1'-c]pyrazine in a hydrocarbon solvent; and then
(b") refluxing the reaction mixture in that selected hydrocarbon solvent under pressure.

9. A process according to claim 8 wherein step (b") is carried out in a time period of from 2 to 6 hours, at a temperature of from 120°C to 160°C, and at a pressure of from 1 to 6 atmospheres.

10. A process according to any preceding claim wherein the reducing agent is selected from the group consisting of dialkyl aluminium hydride, trialkylamine alane complex, or mixtures thereof, wherein the above alkyl groups, the same or different in each occurrence, are selected from straight or branched C₁-C₄ alkyl groups.

11. A process according to claim 10 wherein the reducing agent is selected from the group consisting of diisobutyl aluminium hydride (DIBAL-H), dimethylethylamine alane complex, or mixtures thereof.

12. A process according to claim 11 wherein the reducing agent is diisobutyl aluminium hydride (DIBAL-H).

13. A process according to claim 1 comprising:
a) contacting an aqueous solution of glyoxal to a solution of triethylene tetramine in anhydrous ethanol, by carrying out the reaction in a time period of 1 hour or less so as to obtain decahydrodiimidazo[1,2-a:2',1'-c]pyrazine; and then feeding the reaction crude from step (a) and toluene to a continuous distillation column so as to obtain a solution of decahydrodiimidazo[1,2-a:2',1'-c]pyrazine in toluene; and
b) reducing the thus obtained decahydrodiimidazo[1,2-a:2',1'-c]pyrazine with diisobutyl aluminium hydride (DIBAL-H), wherein step (b) comprises:
(b') adding diisobutyl aluminium hydride (DIBAL-H) to the solution of the decahydrodiimidazo[1,2-a:2',1'-c]pyrazine in toluene at room temperature; and
(b") refluxing the reaction mixture at atmospheric pressure in a period of time of from 18 to 40 hours or, alternatively, refluxing the reaction mixture under pressure of from 1 to 6 atmospheres, in a time period of from 2 to 6 hours, at a temperature of from 120°C to 160°C.

14. A process according to any preceding claim wherein the tetraazamacrocyclic derivative being obtained at the end of step (b) is converted to Cyclen by treatment with sodium fluoride or sodium hydroxide and water.

## Patentansprüche

1. Verfahren zur Herstellung von Cyclen, wobei das Verfahren Folgendes umfasst:
a) Umsetzen von Glyoxal mit Triethylentetramin zum Erhalt von Decahydrodiimidazo[1,2-a:2',1'-c]pyrazin; und
b) Reduzieren des so erhaltenen Decahydrodiimidazo[1,2-a:2',1'-c]pyrazins mit einem Reduktionsmittel aus der Gruppe bestehend aus Dialkylaluminiumhydrid, Trialkylamin-Alan-Komplex oder Mischungen davon;
wobei die obigen Alkylgruppen, die bei jedem Auftreten gleich oder verschieden sind, aus geraden oder verzweigten C₁-C₆-Alkylgruppen ausgewählt sind, und
wobei Schritt (a) durchgeführt wird, indem eine wässrige Lösung von Glyoxal zu einer Lösung des Amins in wasserfreiem Ethanol gegeben wird und indem die Umsetzung in einem Zeitraum von 1 Stunde oder weniger durchgeführt wird.

2. Verfahren nach Anspruch 1, wobei Schritt (a) durchgeführt wird, indem eine wässrige Lösung von Glyoxal zu einer Lösung des Amins in wasserfreiem Ethanol gegeben wird, die unter Raumtemperatur gehalten wird, und indem die Umsetzung unterhalb von Raumtemperatur durchgeführt wird.

3. Verfahren nach Anspruch 2, wobei die Temperatur im Bereich von 0 °C bis 10 °C liegt.

4. Verfahren nach einem der Ansprüche 1 bis 3, wobei am Ende von Schritt (a) ein Lösungsmittelaustausch vorgenommen wird, der das Zuführen des Reaktionsrohprodukts aus Schritt (a) und eines Kohlenwasserstofflösungsmittels zu einer Destillationskolonne umfasst.

5. Verfahren nach Anspruch 4, wobei das Kohlenwasserstofflösungsmittel aus der Gruppe bestehend aus Toluol, Xylol, Ethylbenzol, Mesitylen, Hexan, Heptan, Octan, Isooctan, Nonan, Decan oder Mischungen davon ausgewählt wird.

6. Verfahren nach Anspruch 5, wobei es sich bei dem Kohlenwasserstofflösungsmittel um Toluol handelt.

7. Verfahren nach Anspruch 1, wobei Schritt (b) Folgendes umfasst:
(b') Zugeben des gewählten Dialkylaluminiumhydrids oder Trialkylamin-Alan-Komplexes oder von Mischungen davon zu einer Lösung von Decahydrodiimidazo[1,2-a:2',1'-c]pyrazin in einem Kohlenwasserstofflösungsmittel; und dann
(b") Refluxieren der Reaktionsmischung in diesem gewählten Kohlenwasserstofflösungsmittel bei Normaldruck.

8. Verfahren nach Anspruch 1, wobei Schritt (b) Folgendes umfasst:
(b') Zugeben des gewählten Dialkylaluminiumhydrids oder Trialkylamin-Alan-Komplexes oder von Mischungen davon zu einer Lösung von Decahydrodiimidazo[1,2-a:2',1'-c]pyrazin in einem Kohlenwasserstofflösungsmittel; und dann
(b") Refluxieren der Reaktionsmischung in diesem gewählten Kohlenwasserstofflösungsmittel unter Druck.

9. Verfahren nach Anspruch 8, wobei Schritt (b") in einem Zeitraum von 2 bis 6 Stunden, bei einer Temperatur von 120 °C bis 160 °C und bei einem Druck von 1 bis 6 Atmosphären durchgeführt wird.

10. Verfahren nach einem der vorhergehenden Ansprüche, wobei das Reduktionsmittel aus der Gruppe bestehend aus Dialkylaluminiumhydrid, Trialkylamin-Alan-Komplex oder Mischungen davon ausgewählt wird, wobei die obigen Alkylgruppen, die bei jedem Auftreten gleich oder verschieden sind, aus geraden oder verzweigten C₁-C₄-Alkylgruppen ausgewählt sind.

11. Verfahren nach Anspruch 10, wobei das Reduktionsmittel aus der Gruppe bestehend aus Diisobutylaluminiumhydrid (DIBAL-H), Dimethylethylamin-Alan-Komplex oder Mischungen davon ausgewählt wird.

12. Verfahren nach Anspruch 11, wobei es sich bei dem Reduktionsmittel um Diisobutylaluminiumhydrid (DIBAL-H) handelt.

13. Verfahren nach Anspruch 1, umfassend:
a) Inkontaktbringen einer wässrigen Lösung von Glyoxal mit einer Lösung von Triethylentetramin in wasserfreiem Ethanol durch Durchführen der Umsetzung in einem Zeitraum von 1 Stunde oder weniger zum Erhalt von Decahydrodiimidazo[1,2-a:2',1'-c]pyrazin; und dann Zuführen des Reaktionsrohprodukts aus Schritt (a) und von Toluol zu einer kontinuierlich arbeitenden Destillationskolonne zum Erhalt einer Lösung von Decahydrodiimidazo[1,2-a:2',1'-c]pyrazin in Toluol; und
b) Reduzieren des so erhaltenen Decahydrodiimidazo[1,2-a:2',1'-c]pyrazins mit Diisobutylaluminiumhydrid (DIBAL-H), wobei Schritt (b) Folgendes umfasst:
(b') Zugeben von Diisobutylaluminiumhydrid (DIBAL-H) zu der Lösung des Decahydrodiimidazo[1,2-a:2',1'-c]pyrazins in Toluol bei Raumtemperatur; und
(b") Refluxieren der Reaktionsmischung bei Normaldruck in einem Zeitraum von 18 bis 40 Stunden oder alternativ dazu Refluxieren der Reaktionsmischung bei einem Druck von 1 bis 6 Atmosphären in einem Zeitraum von 2 bis 6 Stunden bei einer Temperatur von 120 °C bis 160 °C.

14. Verfahren nach einem der vorhergehenden Ansprüche, wobei das am Ende von Schritt (b) erhaltene tetraazamacrocyclische Derivat durch Behandlung mit Natriumfluorid oder Natriumhydroxid und Wasser in Cyclen umgewandelt wird.

## Revendications

1. Procédé de préparation de cyclène, lequel procédé comprend :
a) la mise en réaction de glyoxal avec de la triéthylène tétramine de façon à obtenir de la décahydrodiimidazo[1,2-a:2',1'-c]pyrazine ; et
b) la réduction de la décahydrodiimidazo[1,2-a:2',1'-c]pyrazine ainsi obtenue avec un agent réducteur choisi dans le groupe constitué par un hydrure de dialkylaluminium, un complexe de trialkylamine-alane, ou leurs mélanges ;
dans lequel les groupes alkyle ci-dessus, identiques ou différents en chaque occurrence, sont choisis parmi des groupes alkyle en C₁-C₆ linéaires ou ramifiés, et
dans lequel l'étape (a) est effectuée en ajoutant une solution aqueuse de glyoxal à une solution de l'amine dans de l'éthanol anhydre, et en effectuant la réaction en une période de temps de 1 heure ou moins.

2. Procédé selon la revendication 1, dans lequel l'étape (a) est effectuée en ajoutant une solution aqueuse de glyoxal à une solution de l'amine dans de l'éthanol anhydre, maintenue en dessous de la température ambiante, et en effectuant la réaction en dessous de la température ambiante.

3. Procédé selon la revendication 2, dans lequel la température est comprise entre 0 °C et 10 °C.

4. Procédé selon l'une quelconque des revendications 1 à 3, dans lequel, à la fin de l'étape (a), on effectue un changement de solvant comprenant l'alimentation du brut réactionnel provenant de l'étape (a) et d'un solvant hydrocarboné à une colonne de distillation.

5. Procédé selon la revendication 4, dans lequel le solvant hydrocarboné est choisi dans le groupe constitué par le toluène, un xylène, l'éthylbenzène, le mésitylène, l'hexane, l'heptane, l'octane, l'isooctane, le nonane, le décane ou leurs mélanges.

6. Procédé selon la revendication 5, dans lequel le solvant hydrocarboné est le toluène.

7. Procédé selon la revendication 1, dans lequel l'étape (b) comprend :
(b') l'ajout de l'hydrure de dialkylaluminium ou du complexe de trialkylamine alane choisi ou de leurs mélanges à une solution de décahydrodiimidazo[1,2-a:2',1'-c]pyrazine dans un solvant hydrocarboné ; et ensuite
(b") le reflux du mélange réactionnel dans ce solvant hydrocarboné choisi à la pression atmosphérique.

8. Procédé selon la revendication 1, dans lequel l'étape (b) comprend :
(b') l'ajout de l'hydrure de dialkylaluminium ou du complexe de trialkylamine alane choisi ou de leurs mélanges à une solution de décahydrodiimidazo[1,2-a:2',1'-c]pyrazine dans un solvant hydrocarboné ; et ensuite
(b") le reflux du mélange réactionnel dans ce solvant hydrocarboné choisi sous pression.

9. Procédé selon la revendication 8, dans lequel l'étape (b") est effectuée en une période de temps de 2 à 6 heures, à une température de 120 °C à 160 °C, et à une pression de 1 à 6 atmosphères.

10. Procédé selon l'une quelconque des revendications précédentes, dans lequel l'agent réducteur est choisi dans le groupe constitué par un hydrure de dialkylaluminium, un complexe de trialkylamine alane, ou leurs mélanges, dans lequel les groupes alkyle ci-dessus, identiques ou différents en chaque occurrence, sont choisis parmi des groupes alkyle en C₁-C₄ linéaires ou ramifiés.

11. Procédé selon la revendication 10, dans lequel l'agent réducteur est choisi dans le groupe constitué par un hydrure de diisobutylaluminium (DIBAL-H), un complexe de diméthyléthylamine alane, ou leurs mélanges.

12. Procédé selon la revendication 11, dans lequel l'agent réducteur est l'hydrure de diisobutylaluminium (DIBAL-H).

13. Procédé selon la revendication 1, comprenant :
a) la mise en contact d'une solution aqueuse de glyoxal avec une solution de triéthylène tétramine dans de l'éthanol anhydre, en effectuant la réaction en une période de temps de 1 heure ou moins de façon à obtenir de la décahydrodiimidazo[1,2-a:2',1'-c]pyrazine ; et ensuite l'alimentation du brut réactionnel issu de l'étape (a) et de toluène à une colonne de distillation continue de façon à obtenir une solution de décahydrodiimidazo[1,2-a:2',1'-c]pyrazine dans du toluène ; et
b) la réduction de la décahydrodiimidazo[1,2-a:2',1'-c]pyrazine ainsi obtenue avec de l'hydrure de diisobutylaluminium (DIBAL-H), dans lequel l'étape (b) comprend :
(b') l'ajout d'hydrure de diisobutylaluminium (DIBAL-H) à la solution de décahydrodiimidazo[1,2-a:2',1'-c]pyrazine dans du toluène à température ambiante ; et
b") le reflux du mélange réactionnel à pression atmosphérique en une période de temps de 18 à 40 heures ou, en variante, le reflux du mélange réactionnel sous pression de 1 à 6 atmosphères, en une période de temps de 2 à 6 heures, à une température de 120 °C à 160 °C.

14. Procédé selon l'une quelconque des revendications précédentes, dans lequel le dérivé tétraazamacrocyclique obtenu à la fin de l'étape (b) est converti en cyclène par traitement avec du fluorure de sodium ou de l'hydroxyde de sodium et de l'eau.
